# EUROPEAN PATENT APPLICATION

(11) **EP 4 755 912 A1**
(43) Date of publication of application: **10.06.2026**
(21) Application number: 24847696.2
(22) Date of filing: 10.05.2024
(51) Int. Cl.: C07K 16/28, C07K 14/54, C07K 16/18, C07K 16/30, G01N 33/68, G01N 33/577, G01N 33/574

(54) **DIAGNOSTIC ANTIBODY APPLICABLE TO IMMUNOHISTOCHEMISTRY TEST OF CD228**

(30) Priority: 28.07.2023 CN 202310938430
(71) Applicant: Shandong Boan Biotechnology Co., Ltd., Yantai, Shandong 264035 (CN)
(72) Inventor: WANG, Hui, Yantai, Shandong 264035 (CN); SONG, Deyong, Yantai, Shandong 264035 (CN); LI, Min, Yantai, Shandong 264035 (CN); HAN, Jing, Yantai, Shandong 264035 (CN); LIU, Fang, Yantai, Shandong 264035 (CN)
(74) Representative: karo IP
(86) International application number: PCT/CN2024/092159
(87) International publication number: WO 2025/025731

(57) **Abstract**

The present disclosure provides a specific antibody and an antigen-binding fragment thereof against a CD228 protein for use in detecting and treating a variety of tumors, such as melanoma, mesothelioma, and colorectal cancer. An antibody binding fragment facilitates the diagnosis and treatment of cancer by specifically binding to CD228. The present disclosure further comprises antibody-drug conjugates (ADCs) in which a drug binds to an antibody via a linker, aiming to improve the therapeutic effect and reduce the impact on normal cells. Meanwhile, provided is a nucleic acid encoding an antibody or an antigen-binding fragment thereof, so as to produce a recombinant antibody or fragment. In addition, the present disclosure also provides a kit for laboratory research, clinical diagnosis, and therapeutic effect monitoring, which facilitates the determination of whether a tissue or cell expresses CD228 and provides a new strategy for personalized and precise treatment of cancer.

## Description

### TECHNICAL FIELD

The present disclosure relates to the technical field of biomedicine or biopharmaceuticals, and particularly to a novel anti-CD228 antibody, a method for diagnosing cancer using the anti-CD228 antibody, and use of the antibody in preparing a reagent for diagnosing cancer.

### BACKGROUND ART

CD228, also known as melanotransferrin (MTf), p97, and MFI2, is an iron-binding glycoprotein belonging to the transferrin (Tf) molecular family. MTf shares 40% protein sequence identity with human lactoferrin and transferrin and is highly conserved across various species. CD228 plays an indispensable role in iron metabolism in the body.

CD228 was first identified as a surface marker on malignant melanoma cells, is overexpressed in most clinically isolated melanomas, and is also observed in many other human cancers. CD228 is now a broad-spectrum anti-tumor target, showing high expression in cancers such as melanoma, mesothelioma, pancreatic cancer, cervical cancer, gallbladder cancer, and colon cancer. After anti-CD228 antibody drugs bind to CD228, they can inhibit tumor cell proliferation and increase survival rates for tumor patients.

In clinical practice, patients with malignant solid tumors requiring anti-CD228 therapeutic agents must first undergo immunohistochemistry (IHC) detection of tumor tissues to determine whether the tumor tissues express the CD228 molecule and the proportion of cells expressing CD228, thereby enabling more targeted use of anti-CD228 drugs and assisting doctors and patients in rationally selecting treatment modalities.

Immunohistochemistry is the study based on the principle of specific antigen-antibody binding to localize, qualify, and relatively quantify antigens within tissues. The tissue sections used in immunohistochemistry include two types: paraffin sections and frozen sections. Both types of tissue sections have their own advantages and disadvantages: Paraffin sections have the advantages of well-preserved tissue structure, ability to be cut into continuous thin slices, clear tissue structure, accurate antigen localization, and long-term storage; the disadvantage is that during the preparation process, they are treated with organic solvents such as alcohol and xylene, resulting in significant loss of antigen activity within the tissue, which affects the conformation of the antigen and the exposure of antigenic epitopes. Frozen sections have the advantage of better preserving the immunological activity of various antigens, especially cell surface antigens for which frozen sections should be used; the disadvantages are that the tissue is fragile and prone to damage, obtaining intact section structures is difficult, and water in the tissue easily forms ice crystals during freezing, which often affects antigen localization, so frozen sections are often used for intraoperative rapid pathological diagnosis and are only suitable for short-term low-temperature storage.

During the development of immunohistochemistry detection methods, there are currently two main drawbacks: one is that the antigenic epitopes exposed by the antigen in the tissue section may differ from those in the native state, leading to insufficient affinity and specificity of the detection antibody and low detection sensitivity; the other is the presence of non-specific binding of the detection antibody in various types of normal tissues and tumor tissues in the human body, leading to false positive results. For the reasons described above, current clinical immunohistochemistry detection requires first screening indications and control antibodies, then optimizing the experimental system, using a large number of tests to screen suitable diagnostic antibodies from numerous candidate antibodies, and evaluating indicators such as the sensitivity, specificity, and accuracy of the candidate antibodies in immunohistochemistry detection using different tissue paraffin sections or frozen sections.

Therefore, an effective, rapid, and low-cost diagnostic test to detect CD228 in tissues is highly necessary for clinical diagnosis and targeted therapy.

### SUMMARY

The present disclosure provides an anti-CD228 antibody or an antigen-binding fragment thereof. The anti-CD228 antibody provided in the present disclosure has a relatively high binding ability to CD228 and is suitable for diagnosis using immunohistochemistry detection. In immunohistochemistry detection, the anti-CD228 antibody provided in the present disclosure can achieve a staining effect similar to that of existing commercial anti-CD228 antibodies at working concentrations, and can replace existing commercial anti-CD228 monoclonal antibodies used in immunohistochemistry.

A first aspect of the present disclosure provides an antibody or an antigen-binding fragment thereof specifically binding to a CD228 protein, and the antibody or the antigen-binding fragment thereof comprises the following 3 light chain complementarity determining regions and 3 heavy chain complementarity determining regions, wherein
the 3 light chain complementarity determining regions of the antibody or the antigen-binding fragment thereof comprise an LCDR1 set forth in SEQ ID NO: 2, an LCDR2 set forth in SEQ ID NO: 3, and an LCDR3 set forth in SEQ ID NO: 4; and the 3 heavy chain complementarity determining regions comprise an HCDR1 set forth in SEQ ID NO: 6, an HCDR2 set forth in SEQ ID NO: 7, and an HCDR3 set forth in SEQ ID NO: 8;
the 3 light chain complementarity determining regions of the antibody or the antigen-binding fragment thereof comprise an LCDR1 set forth in SEQ ID NO: 10, an LCDR2 set forth in SEQ ID NO: 11, and an LCDR3 set forth in SEQ ID NO: 12; and the 3 heavy chain complementarity determining regions comprise an HCDR1 set forth in SEQ ID NO: 14, an HCDR2 set forth in SEQ ID NO: 15, and an HCDR3 set forth in SEQ ID NO: 16;
or the 3 light chain complementarity determining regions of the antibody or the antigen-binding fragment thereof comprise an LCDR1 set forth in SEQ ID NO: 18, an LCDR2 set forth in SEQ ID NO: 19, and an LCDR3 set forth in SEQ ID NO: 20; and the 3 heavy chain complementarity determining regions comprise an HCDR1 set forth in SEQ ID NO: 22, an HCDR2 set forth in SEQ ID NO: 23, and an HCDR3 set forth in SEQ ID NO: 24.

Further, the antibody or the antigen-binding fragment thereof comprises a light chain variable region having at least 95%, 96%, 97%, 98%, 99%, or 100% identity to the amino acid sequence set forth in SEQ ID NO: 1, and a heavy chain variable region having at least 95%, 96%, 97%, 98%, 99%, or 100% identity to the amino acid sequence set forth in SEQ ID NO: 5;
the antibody or the antigen-binding fragment thereof comprises a light chain variable region having at least 95%, 96%, 97%, 98%, 99%, or 100% identity to the amino acid sequence set forth in SEQ ID NO: 9, and a heavy chain variable region having at least 95%, 96%, 97%, 98%, 99%, or 100% identity to the amino acid sequence set forth in SEQ ID NO: 13;
or the antibody or the antigen-binding fragment thereof comprises a light chain variable region having at least 95%, 96%, 97%, 98%, 99%, or 100% identity to the amino acid sequence set forth in SEQ ID NO: 17, and a heavy chain variable region having at least 95%, 96%, 97%, 98%, 99%, or 100% identity to the amino acid sequence set forth in SEQ ID NO: 21.

Further, the antibody or the antigen-binding fragment thereof comprises a heavy chain constant region having at least 95%, 96%, 97%, 98%, 99%, or 100% identity to the amino acid sequence set forth in SEQ ID NO: 25, and comprises a light chain constant region having at least 95%, 96%, 97%, 98%, 99%, or 100% identity to the amino acid sequence set forth in SEQ ID NO: 26.

In embodiments of the present disclosure, the antibody or the antigen-binding fragment thereof of the present disclosure includes a monoclonal antibody, a polyclonal antibody, a chimeric antibody, a humanized antibody, a Fab, a Fab', a F(ab')2, an Fv, an scFv, a dsFv fragment, or the like.

A second aspect of the present disclosure provides a nucleic acid encoding the antibody or the antigen-binding fragment thereof.

A third aspect of the present disclosure provides a vector comprising the nucleic acid encoding the antibody or the antigen-binding fragment thereof. The vector can be used to express the antibody or the antigen-binding fragment thereof. Preferably, the vector may be a viral vector; preferably, the viral vector includes, but is not limited to, a lentivirus vector, an adenovirus vector, an adenoassociated virus vector, a retrovirus vector, or the like; preferably, the vector may be a non-viral vector; preferably, the vector may be a mammalian cell expression vector; preferably, the expression vector may be a bacterial expression vector; preferably, the expression vector may be a fungal expression vector.

A fourth aspect of the present disclosure provides a cell, and the cell comprises the nucleic acid or the vector and is capable of expressing the antibody or the antigen-binding fragment thereof. Preferably, the cell is a bacterial cell; preferably, the bacterial cell is an *Escherichia coli* cell or the like; preferably, the cell is a fungal cell; preferably, the fungal cell is a yeast cell; preferably, the yeast cell is a *Pichia pastoris* cell or the like; preferably, the cell is a mammalian cell; preferably, the mammalian cell is a Chinese hamster ovary (CHO) cell, a human embryonic kidney cell (293), a B cell, a T cell, a DC cell, an NK cell, or the like.

A fifth aspect of the present disclosure provides an antibody conjugate, and the antibody conjugate consists of the antibody or the antigen-binding fragment thereof described in the present disclosure conjugated to at least one detectable marker.

A sixth aspect of the present disclosure provides a kit, which comprises the antibody or the antigen-binding fragment thereof described in the present disclosure, the conjugate described in the present disclosure, or the nucleic acid encoding the antibody or the antigen-binding fragment thereof described in the present disclosure; optionally, the kit comprises a buffer reagent for detection.

A seventh aspect of the present disclosure provides a kit for detecting or quantifying CD228, and the kit comprises the anti-CD228 antibody or the antigen-binding fragment thereof described in the present disclosure or the conjugate described in the present disclosure; optionally, the kit further comprises a related buffer solution for detection or quantification.

An eighth aspect of the present disclosure provides a kit for diagnosing a disease or evaluating the therapeutic effect on a disease, and the kit comprises the antibody or the antigen-binding fragment thereof specifically binding to a CD228 protein described in the present disclosure, wherein the disease is a CD228-related disease.

A ninth aspect of the present disclosure provides use of the anti-CD228 antibody or the antigen-binding fragment thereof, the coding nucleic acid, the vector, or the cell described in the present disclosure in preparing a kit for detecting and/or quantifying whether a cell or tissue expresses CD228, and the use is for a non-diagnostic purpose and/or a non-predictive purpose for the therapeutic effect on a disease.

Preferably, the kit is an immunohistochemistry detection kit.

A tenth aspect of the present disclosure provides use of the antibody or the antigen-binding fragment thereof specifically binding to a CD228 protein, the coding nucleic acid, the vector, or the cell described in the present disclosure in preparing a kit for diagnosing a disease, wherein the disease is a CD228-related disease;
preferably, the kit is an immunohistochemistry detection kit;
preferably, the CD228-related disease includes one or more of melanoma, lung cancer, gastric cancer, colon cancer, mesothelioma, pancreatic cancer, breast cancer, esophageal cancer, cervical cancer, gallbladder cancer, and cholangiocarcinoma.

An eleventh aspect of the present disclosure provides use of the anti-CD228 antibody or the antigen-binding fragment thereof, the coding nucleic acid, the vector, or the cell described in the present disclosure in preparing a kit for evaluating the therapeutic effect on a CD228-related disease, wherein the disease is a CD228-related disease;
preferably, the kit is an immunohistochemistry detection kit;
preferably, the CD228-related disease includes one or more of melanoma, lung cancer, gastric cancer, colon cancer, mesothelioma, pancreatic cancer, breast cancer, esophageal cancer, cervical cancer, gallbladder cancer, and cholangiocarcinoma.

A twelfth aspect of the present disclosure provided a method for detecting or quantifying an anti-CD228 antibody in a sample, and the method is for diagnostic or non-diagnostic purposes and comprises: step (i) contacting the anti-CD228 antibody or the antigen-binding fragment thereof, the coding nucleic acid, the vector, the cell, or the antibody conjugate described in the present disclosure with a sample to be tested; and (ii) detecting a complex formed via the binding of the antibody or the antigen-binding fragment thereof to CD228 expressed in the sample, or detecting a complex formed via the binding of the antibody conjugate to CD228 expressed in the sample.

Further, the detection and/or quantification method is an immunohistochemistry method, and the method comprises the step of performing immunohistochemistry detection on a sample using the anti-CD228 antibody or the antigen-binding fragment thereof, the coding nucleic acid, the vector, or the cell described in the present disclosure.

A thirteenth aspect of the present disclosure provides a method for diagnosing and monitoring cancer, which comprises: step (i) contacting a sample with the anti-CD228 antibody or the antigen-binding fragment thereof described in the present disclosure or the antibody conjugate described in the present disclosure, and (ii) detecting a complex formed via the binding of the antibody or the antigen-binding fragment thereof to CD228 expressed in the sample, or detecting a complex formed via the binding of the antibody conjugate to CD228 expressed in the sample.

The disease is a CD228-related disease, and preferably, the CD228-related disease includes one or more of melanoma, lung cancer, gastric cancer, colon cancer, mesothelioma, pancreatic cancer, breast cancer, esophageal cancer, cervical cancer, gallbladder cancer, and cholangiocarcinoma.

A fourteenth aspect of the present disclosure provides a method for evaluating the therapeutic effect on a disease, wherein the disease is a CD228-related disease. The method comprises: step (i) contacting a sample with the anti-CD228 antibody or the antigen-binding fragment thereof described in the present disclosure or the antibody conjugate described in the present disclosure, and (ii) detecting a complex formed via the binding of the antibody or the antigen-binding fragment thereof to CD228 expressed in the sample, or detecting a complex formed via the binding of the antibody conjugate to CD228 expressed in the sample.

Further, the CD228-related disease includes one or more of melanoma, lung cancer, gastric cancer, colon cancer, mesothelioma, pancreatic cancer, breast cancer, esophageal cancer, cervical cancer, gallbladder cancer, and cholangiocarcinoma.

Further, the evaluation method is a method for determining whether the disease can be treated by a cancer therapy targeting CD228.

A fifteenth aspect of the present disclosure provides an immunohistochemistry detection method using the anti-CD228 antibody of the present disclosure, and the method comprises: 1) preparing a tissue section; 2) applying the anti-CD228 antibody as a primary antibody to the prepared tissue section, and incubating for 30 min; 3) adding a second antibody binding to the first antibody, labeling, developing color, washing, and mounting; 4) observing under a microscope and detecting. Further, the tissue section is one or more of melanoma, lung cancer, gastric cancer, colon cancer, mesothelioma, pancreatic cancer, breast cancer, esophageal cancer, cervical cancer, gallbladder cancer, and cholangiocarcinoma.

Further, the use concentration of the diagnostic antibody, namely the anti-CD228 antibody, in the detection of human cancer tissues is 1 µg/mL-4 µg/mL, preferably 1 µg/mL, 2 µg/mL, 3 µg/mL, or 4 µg/mL.

The present disclosure has the following beneficial effects:
1. Sensitivity and affinity: Through ELISA and bio-layer interferometry (BLI) experiments, the anti-CD228 antibody of the present disclosure exhibits high sensitivity and strong affinity for the CD228 protein.
2. Specific binding: The anti-CD228 antibody of the present disclosure specifically binds only to the CD228 protein, and has substantially no binding to transferrin (TF) and lactoferrin (LF) of the same family, showing good selectivity and specificity.
3. Staining effect: In immunohistochemistry (IHC) staining of various cancer tissues, the staining effect of the anti-CD228 antibody of the present disclosure is comparable to or superior to that of the commercial antibody NBP1-85777. Particularly in the detection of certain specific cancer types, the positive rate and staining sensitivity of the anti-CD228 antibody of the present disclosure are significantly higher than those of NBP1-85777.
4. Applicability: The anti-CD228 antibody of the present disclosure shows consistent staining intensities and positive rates in tissue chips of various cancer types (melanoma, lung cancer, gastric cancer, colon cancer, mesothelioma, pancreatic cancer, breast cancer, esophageal cancer, cervical cancer, gallbladder cancer, and cholangiocarcinoma), indicating its broad applicability.
5. Stability: The anti-CD228 antibody of the present disclosure exhibits good stability under different environmental conditions.
6. Substitutability: Based on the experimental results described above, the anti-CD228 antibody of the present disclosure can serve as an effective substitute for the commercial antibody NBP1-85777 in immunohistochemistry detection of the CD228 target. Early discovery and treatment selection: Due to the high positive rate and specificity of the anti-CD228 antibody of the present disclosure, the antibody can be used for early detection and more precise treatment selection for cancer.

### BRIEF DESCRIPTION OF THE DRAWINGS

FIG. 1 shows the serum titers of CD228-immunized mice.
FIG. 2 shows the serum titers of CD228-immunized rabbits.
FIG. 3 shows the results for the IHC detection of CD228-immunized rabbit serum.
FIG. 4 shows the results for the binding sensitivity of each rabbit anti-CD228 monoclonal antibody to the CD228 protein detected by ELISA.
FIG. 5 shows the bio-layer interferometry (BLI) detection spectrum for the affinity between CA-2204 and CD228.
FIG. 6 shows the bio-layer interferometry (BLI) detection spectrum for the affinity between R546 and CD228.
FIG. 7 shows the bio-layer interferometry (BLI) detection spectrum for the affinity between R526 and CD228.
FIG. 8 shows the results for the binding specificity of rabbit anti-CD228 monoclonal antibodies to the CD228 protein detected by Western Blot.
FIG. 9 shows the flowchart of the Leica BOND^{™} fully automated IHC and ISH staining system.
FIG. 10 shows the results for the screening of positive and negative tissues with the CD228 control antibody.
FIG. 11 shows comparative images of the staining effects of different commercial antibodies in different tissues.
FIG. 12 shows the comparison of the staining of candidate antibodies at different concentrations in the same mesothelioma tissue.
FIG. 13 shows images with the results of DAB staining for the R546 antibody in mesothelioma tissues at 1 min and 5 min.
FIG. 14 shows the comparison of the staining effects of candidate anti-CD228 antibodies and the commercial antibody in mesothelioma tissues.
FIG. 15 shows the comparison of the staining effects of R546 and the commercial antibody in paraffin-embedded tissue sections.
FIG. 16 shows the comparison of the staining sensitivity and specificity of R546 and NBP1-85777 in colon cancer tissue chips.
FIG. 17 shows the comparison of the staining sensitivity and specificity of R546 and NBP1-85777 in cervical cancer tissue chips.
FIG. 18 shows the comparison of the staining sensitivity and specificity of R546 and NBP1-85777 in esophageal cancer tissue chips.
FIG. 19 shows the comparison of the staining sensitivity and specificity of R546 and NBP1-85777 in pancreatic cancer tissue chips.
FIG. 20 shows the comparison of the staining sensitivity and specificity of R546 and NBP1-85777 in gastric cancer tissue chips.
FIG. 21 shows the comparison of the staining sensitivity and specificity of R546 and NBP1-85777 in extrahepatic cholangiocarcinoma tissue chips.
FIG. 22 shows the comparison of the staining of R546 and NBP1-85777 in gallbladder cancer tissue chips.
FIG. 23 shows the comparison of the staining in individual tissue sections of gallbladder cancer between R546 and NBP1-85777.
FIG. 24 shows the comparison of the staining in individual tissue sections of cholangiocarcinoma between R546 and NBP1-85777.
FIG. 25 shows the comparison of the staining in individual tissue sections of esophageal cancer between R546 and NBP1-85777.
FIG. 26 shows the comparison of the staining in individual tissue sections of pancreatic cancer between R546 and NBP1-85777.
FIG. 27 shows the comparison of the staining in individual tissue sections of gastric cancer between R546 and NBP1-85777.
FIG. 28 shows the results for the binding sensitivity detection of R546 and NBP1-85777 to CD228 and other proteins of the same family detected by ELISA.
FIG. 29 shows the bio-layer interferometry detection spectra for the affinity of R546 and NBP1-85777 for CD228 and other proteins of the same family.

### DETAILED DESCRIPTION

### Definitions

For a clearer understanding of the present disclosure, certain terms are first defined herein. As used in the present application, unless otherwise expressly provided herein, the following terms shall have the meanings set forth below. Additional definitions are set forth throughout the application. Terms used herein: An antibody and an antigen-binding fragment thereof may be immunoglobulins (Igs), monoclonal antibodies (mAbs), polyclonal antibodies (pAbs), antibody fragments, singlechain variable regions (scFvs), etc. An antibody and an antigen-binding fragment thereof refer to an antibody or a derivative thereof capable of binding to an antigen, which holds significant applications in biomedical research, diagnosis, and treatment. Monoclonal antibodies and polyclonal antibodies are two primary types of antibodies. The former are produced by a single clone and exhibit high specificity to a single antigenic epitope; the latter are produced by multiple clones and can recognize multiple antigenic epitopes. Antibody fragments, such as scFvs, are smaller portions of antibodies that retain specific antigen-binding capability while possessing a simpler structure.

Terms used herein: Immunohistochemistry detection may be immunohistochemistry (IHC), immunofluorescence (IF), immunoperoxidase staining (peroxidase-based IHC), immunogold staining, etc. Immunohistochemistry detection is a technique that utilizes the principle of specific binding between antibodies and specific antigens to localize, qualify, and quantify antigens in tissue sections or cell samples. Immunofluorescence and immunoperoxidase staining are common methods in immunohistochemistry detection, which visualize the location and expression level of antigens through fluorescent labeling and enzyme labeling, respectively. Immunogold staining uses gold nanoparticles as labels, providing high-resolution antigen localization.

Terms used herein: Cancer refers to a group of diseases characterized by abnormal proliferation and uncontrolled growth of cells, where such abnormal cells possess the ability to invade surrounding tissues and metastasize to distant sites via the blood and lymphatic systems. A malignant tumor is a type of cancer characterized by rapid growth, strong resistance to treatment, and a high risk of recurrence and metastasis. Cancer lesions and tumor pathology refer to the manifestations and characteristics of cancer at the tissue and cellular levels.

Terms used herein: Therapeutic effect evaluation refers to therapeutic efficacy assessment, treatment response monitoring, clinical efficacy analysis, or treatment outcome determination.

Therapeutic effect evaluation refers to the assessment of the impact of medical intervention measures on the disease process through a series of biomarkers, imaging examinations, clinical symptoms, and laboratory indicators. This includes comparative analysis before and after treatment, as well as long-term follow-up of therapeutic effects. Therapeutic effect evaluation is of significant importance for guiding clinical treatment decisions, optimizing treatment regimens, and improving patients' quality of life.

### Example 1: Production of Anti-CD228 Monoclonal Antibodies

### 1. Experimental method

### (1) Preparation of immunogen

**Table 1. Amino acid sequence of human CD228 protein**

| Protein ID | Amino acid sequence |
|---|---|
| Human CD228 | MRGPSGALWLLLALRTVLGGMEVRWCATSDPEQHKCGNMSEAFREAGIQPS LLCVRGTSADHCVQLIAAQEADAITLDGGAIYEAGKEHGLKPVVGEVYDQEV GTSYYAVAVVRRSSHVTIDTLKGVKSCHTGINRTVGWNVPVGYLVESGRLSV MGCDVLKAVSDYFGGSCVPGAGETSYSESLCRLCRGDSSGEGVCDKSPLERY YDYSGAFRCLAEGAGDVAFVKHSTVLENTDGKTLPSWGQALLSQDFELLCR DGSRADVTEWRQCHLARVPAHAVVVRADTDGGLIFRLLNEGQRLFSHEGSSF QMFSSEAYGQKDLLFKDSTSELVPIATQTYEAWLGHEYLHAMKGLLCDPNRL PPYLRWCVLSTPEIQKCGDMAVAFRRQRLKPEIQCVSAKSPQHCMERIQAEQ VDAVTLSGEDIYTAGKTYGLVPAAGEHYAPEDSSNSYYVVAVVRRDSSHAFT LDELRGKRSCHAGFGSPAGWDVPVGALIQRGFIRPKDCDVLTAVSEFFNASC VPVNNPKNYPSSLCALCVGDEQGRNKCVGNSQERYYGYRGAFRCLVENAGD VAFVRHTTVFDNTNGHNSEPWAAELRSEDYELLCPNGARAEVSQFAACNLA QIPPHAVMVRPDTNIFTVYGLLDKAQDLFGDDHNKNGFKMFDSSNYHGQDL LFKDATVRAVPVGEKTTYRGWLGLDYVAALEGMSSQQHHHHHHHHHH(SE Q ID NO:27) |

### (2) Immunization of animals

### A) Immunization of mice

The mice used for immunization experiments were wild-type Balb/c mice. The immunization was performed using a CD228 antigenic protein (0.23 mg/mL, Boan, 20200924, SEQ ID NO: 27) produced by Shandong Boan Biotechnology Co., Ltd.

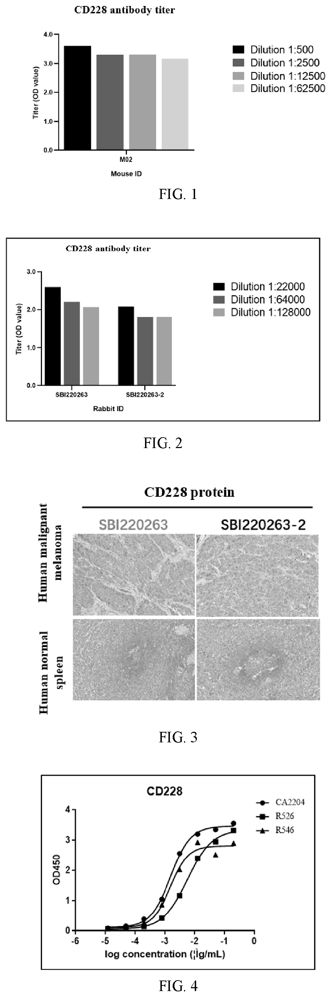

The immunization was performed by multipoint subcutaneous injections in the abdominal and inguinal areas, with an antigenic protein immunization dose of 20 µg/mouse. The antigen was emulsified with complete Freund's adjuvant for the first immunization and with incomplete Freund's adjuvant for the second to fourth immunizations. The first batch of mice received 3 immunizations and one booster immunization, while the second batch of mice received 4 immunizations and one booster immunization. The interval between each immunization was 14 days. Starting from the second immunization, serum was collected from the peripheral blood on day 7 after each immunization to measure antibody titers, and mice with unqualified titers were excluded. After the mice were immunized, the results of the serum titer detection are shown in FIG. 1, where 2500X, 12500X, and 62500X represent the dilution ratios. 3 days after the booster immunization, the mice were euthanized, and the spleen was collected and prepared into single cells for library construction.

### B) Immunization of rabbits

The rabbit immunization experiment was commissioned to Beijing Sino Biological Inc. 2 Japanese white rabbits were used. The immunization was performed using a CD228 antigenic protein (0.23 mg/mL, Boan, 20200924, SEQ ID NO: 27) produced by Shandong Boan Biotechnology Co., Ltd. Each rabbit was immunized with the antigen at a dose of 500 µg. For the first immunization, the immunogen and an equal volume of complete Freund's adjuvant were prepared into an emulsifier, which was administered via multipoint subcutaneous injections on the back. At intervals of 2 weeks, the same dose of immunogen and an equal volume of incomplete Freund's adjuvant were prepared into an emulsifier. One week after the third immunization, blood was collected, and serum titers were determined by ELISA. Rabbits with qualified serum titers had their spleens and bone marrow harvested and frozen in liquid nitrogen for later use. The serum was sent for IHC detection, and animals with favorable IHC results were selected for library construction.

Serum was diluted at ratios of 1:22000, 1:64000, and 1:128000. Serum titer results detected by ELISA are shown in FIG. 2. Simultaneously, serum results detected by IHC (melanoma tissue) are shown in FIG. 3. 1 rabbit with the optimal titer and IHC detection results was selected for library construction and screening.

### C) Library construction and screening

Based on the serum titer and IHC detection results, 1 rabbit and 1 mouse exhibiting the best immune response were selected for library construction. Positive clones were screened by ELISA for subsequent recombinant expression production.

### 1) Phage library construction:

According to the serum titer detection results, the spleens from 1 rabbit and 1 mouse with the best efficacy were selected for RNA extraction. The antibody variable region genes were amplified by RT-PCR. The target heavy and light chain fragments were then spliced to obtain fused scFv fragments, which were inserted into an expression vector. Competent cells were electroporated to obtain the phage antibody library. The library capacity data of the constructed libraries are shown in Table 2-Table 3.

**Table 2. Phage library capacity constructed from immunized mice**

| | |
|---|---|
| Library name | M2 |
| Library capacity (n) | 1.2x10⁹ |

**Table 3. Phage library capacity constructed from immunized rabbits**

| | |
|---|---|
| Library name | 22LY58-3 |
| Library capacity (n) | 7.5E8 |

### 2) Phage library screening:

A plate was coated with a CD228-His protein (self-made). The next day, the phage library was added, and the mixture was incubated for 2 h. After 4-10 times of washing, specifically bound phages were eluted with an elution buffer. The antibody clones obtained by screening and their sources are listed in Table 4 and Table 5.

**Table 4. Source of anti-CD228 antibody obtained from screening**

| Clone | Source library | Source mouse |
|---|---|---|
| CA2204 | CD228-M2 | M2 |

**Table 5. Source of anti-CD228 antibodies obtained from screening**

| Clone | Source library | Source rabbit |
|---|---|---|
| R526 | 22LY58-3 | SBI220263 |
| R546 | 22LY58-3 | SBI220263-2 |

### 3) Detection of positive clones:

The positive library was plated to pick monoclones for expression. The expression supernatant was subjected to ELISA detection, and clones binding to the antigen were selected as positive clones. Positive clones were subjected to PCR and enzyme digestion typing detection. Clones with different banding patterns were selected for ELISA re-detection. After confirming they were positive, they were sent for sequencing.

### 4) Sequence confirmation and expression vector construction:

Positive clones with different sequences were selected for full-length antibody expression vector construction. After confirming the sequences were correct, they were transferred for expression production.

### D) Molecular construction and production of intact antibodies

The antibody variable region genes were amplified by the conventional molecular biology technique PCR (2× Phanta Max Master Mix, manufacturer: Vazyme, Cat. No.: P515-P1-AA). The antibody heavy chain variable region genes were ligated into the vector pCDNA3.4 (Life Technology) having the nucleic acid sequence of the rabbit antibody heavy chain constant region sequence via homologous recombination. The antibody light chain variable region genes were ligated into the vector pCDNA3.4 (Life Technology) having the nucleic acid sequence of the rabbit antibody light chain constant region sequence via homologous recombination. HEK293 cells were co-transfected with plasmids extracted from the sequenced positive clones and incubated on a shaker at 37 °C/8% CO2/125 rpm. After 7 days of the transient expression, the supernatant was purified by Protein A affinity chromatography to obtain the antibodies. The antibody concentration was determined by UV280 in conjunction with the theoretical extinction coefficient. The amino acid sequences of the heavy and light chain constant regions of the three antibodies are shown in Table 6. The amino acid sequences of the variable regions of the three antibodies are shown in Table 7 (CDR regions are underlined, and the analysis system is the IMGT system).

### 2. Experimental results

FIG. 1 shows the serum titer experimental results of mice immunized using the method of this example;
FIG. 2 shows the serum titer experimental results of rabbits immunized using the method of this example;
FIG. 3 shows the IHC detection results of the serum from CD228-immunized rabbits using the method of this example;

The amino acid sequences of the heavy and light chain constant regions of the three antibodies obtained by screening in this example are shown in Table 6. The amino acid sequences of the variable regions of the three antibodies are shown in Table 7 (CDR regions are underlined, and the analysis system is the IMGT system).

**Table 6. Amino acid sequences of heavy and light chain constant regions of 3 antibodies**

| |
|---|
| Heavy chain constant region sequence: |
| GQPKAPSVFPLAPCCGDTPSSTVTLGCLVKGYLPEPVTVTWNSGTLTNGVRTFPSVRQS SGLYSLSSVVSVTSSSQPVTCNVAHPATNTKVDKTVAPSTCSKPTCPPPELLGGPSVFIF PPKPKDTLMISRTPEVTCVVVDVSQDDPEVQFTWYINNEQVRTARPPLREQQFNSTIRV VSTLPIAHQDWLRGKEFKCKVHNKALPAPIEKTISKARGQPLEPKVYTMGPPREELSSR SVSLTCMINGFYPSDISVEWEKNGKAEDNYKTTPAVLDSDGSYFLYSKLSVPTSEWQR GDVFTCSVMHEALHNHYTQKSISRSPGK (SEQ ID NO:25) |
| Light chain constant region: |
| GDPVAPTVLIFPPAADQVATGTVTIVCVANKYFPDVTVTWEVDGTTQTTGIENSKTPQ NSADCTYNLSSTLTLTSTQYNSHKEYTCKVTQGTTSVVQSFNRGDC (SEQ ID NO:26) |

**Table 7. Amino acid sequences of variable regions of 3 antibodies**

| Antibody ID | Light chain variable region sequence | Heavy chain variable region sequence |
|---|---|---|
| R546 | DPMLTQTASPVSAAVGSTVTINCQAS QSVYNNNNLNWYQQKPGQPPKLLIY AASTLASGVPSRFKGSGSGTQFTLTIS DVQCDDAATYYCLGAYDCSSADCG AFGGGTELEIL(SEQ ID NO:1) | QSLEESGGRLVKPDETLTLTCTVSGF SLSSYDMSWVRQAPGKGLQWIGAA TAGGNTYHASWAKGRFTISKTSTTV DLKITSPTTEDTATYFCTRGLNIWGP GTLVTVSS(SEQ ID NO:5) |
| | CDR | CDR |
| | QSVYNNNN(SEQ ID NO:2) | GFSLSSYD(SEQ ID NO:6) |
| | AAS(SEQ ID NO:3) | ATAGGNT(SEQ ID NO:7) |
| | LGAYDCSSADCGA(SEQ ID NO:4) | TRGLNT(SEQ ID NO:8) |
| R526 | DPVLTQTASPVSAAVGGTITINCQAS QSVYNNNQLSWFQQNPGQPPKRLIHS TSTLAPGVSSRFKGSGSGTQFTLTISD VQCDDAATYYCLGSYDCVSADCNAF GGGTEVVVK(SEQ ID NO:9) | QEQLEESGGRLVTPGTPLTLTCTVSG FSLSTYAINWIRQAPGKGLEWIGIIGG NGATYYTNWAKGRFTISKTSTTVDL KITSPTIEDTATYFCARGTNFWGPGT LVTVSS(SEQ ID NO:13) |
| | CDR | CDR |
| | QSVYNNNQ(SEQ ID NO:10) | GFTFSSYA(SEQ ID NO:14) |
| | STS(SEQ ID NO:11) | IGGNGAT(SEQ ID NO:15) |
| | LGSYDCVSADCNA(SEQ ID NO:12) | ARGTNF(SEQ ID NO:16) |
| CA2204 | DVLMTQTPLTLSVTIGQPASISCKSSQ SLLDSDGKTYLNWLLQRPGQSPKRLI YLVSKLDSGVPDRFTGSGSGTDFTLK ISRVEAEDLGVYYCWQGTHFPRTFG GGTKLELK(SEQ ID NO:17) | EVQLQQSGAELVRPGTSVKVSCKAS GYAFTNYLIEWIKQRPGQGLEWIGMI NPGSGGTKYNEKFKGKATLTADKSS STAYMQLSSLTSDDSAVYFCARDYY GYYWGQGTTLTVSS(SEQ ID NO:21) |
| | CDR | CDR |
| | QSLLDSDGKTY(SEQ ID NO:18) | GYAFTNYL(SEQ ID NO:22) |
| | LVS(SEQ ID NO:19) | INPGSGGT(SEQ ID NO:23) |
| | WQGTHFPRT(SEQ ID NO:20) | ARDYYGYY(SEQ ID NO:24) |

### Example 2: Detection of Binding Sensitivity of Rabbit Anti-CD228 Monoclonal Antibodies to CD228 Protein

### 1. Experimental method

The CD228 protein was diluted to 0.2 µg/mL with CBS and added to a plate at 100 µL/well, and the plate was incubated at 4 °C overnight. A blocking solution was added, and the plate was blocked at 37 °C for 1 h and washed. The rabbit CD228 antibody was subjected to a dilution (starting from 0.2 µg/mL, four-fold dilution, 8 gradients) with PBST and added to the plate at 100 µL/well, and the plate was incubated at 37 °C for 1 h and washed. The enzyme-labeled secondary antibody, Goat pAb to Rb IgG (HRP), was added at 100 µL/well, and the plate was incubated at 37 °C for 1 h and washed. 100 µL of a TMB chromogenic solution was added for color development for 10 min, and 2 M sulfuric acid was added to stop the reaction. The OD450nm values were read using a microplate reader.

### 2. Experimental results

The OD450 values were plotted against the antibody concentrations, and the results are shown in FIG. 4. The EC50 (half maximal effective concentration) values for each monoclonal antibody were obtained by calculation based on the data in the figure, as shown in Table 8.

**Table 8. EC50 of rabbit anti-CD228 monoclonal antibodies binding to CD228 protein**

| Serial number | Antibody name | EC50 (ng/mL) |
|---|---|---|
| 1 | R546 | 1.524 |
| 2 | CA2204 | 1.536 |
| 3 | R526 | 5.706 |

As can be seen from the data in Table 8, antibodies 546, 526, and 2204 can all bind to the CD228 protein.

FIG. 4 shows the results for the binding sensitivity of each rabbit anti-CD228 monoclonal antibody to the CD228 protein detected by ELISA according to the present application.

### Example 3: Detection of Affinity of Rabbit Anti-CD228 Monoclonal Antibodies for CD228 by Bio-Layer Interferometry (BLI)

### 1. Experimental method

The rabbit antibodies were coupled to ProA (Fortebio, Cat. No.: 18-5010) chips on the Fortebio RED96 instrument at a concentration of 4 µg/mL, with a threshold set to 1.5 nm. The CD228 protein was subjected to a 2-fold gradient dilution starting from 100 nM with PBST to obtain 4 concentrations. The association lasted for 150 s, the dissociation lasted for 300 s, the equilibrium lasted for 60 s, and the probe regeneration lasted for 15 s. Kinetic parameters, including the KD value, were calculated using the instrument's supporting software.

### 2. Experimental results

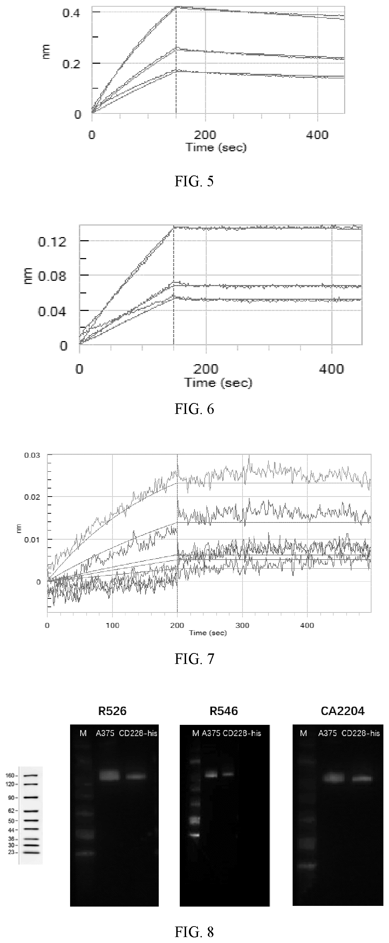

The kinetic parameters, including the KD value, were calculated using the instrument's supporting software in this example. The detection data are shown in Table 9. The affinity detection spectra for the anti-CD228 antibodies CA2204, R546, and R526 obtained by screening in this project are shown in FIG. 5, FIG. 6, and FIG. 7, respectively. Specifically, FIG. 5 shows the bio-layer interferometry (BLI) detection spectrum for the affinity between CA-2204 and CD228 in this example; FIG. 6 shows the bio-layer interferometry (BLI) detection spectrum for the affinity between R546 and CD228 in this example; FIG. 7 shows the bio-layer interferometry (BLI) detection spectrum for the affinity between R526 and CD228 in this example.

**Table 9. KD values of rabbit anti-CD228 monoclonal antibodies binding to CD228 protein**

| Serial number | Clone name | KD (M) | Response |
|---|---|---|---|
| 1 | R546 | 1.33E-09 | 0.1278 |
| 2 | CA2204 | 8.52E-09 | 0.4009 |
| 3 | R526 | <1.0E-12 | 0.0251 |

The spectra shown in FIGs. 5-7 and the data in Table 9 indicate that only the R546 and CA2204 antibodies can bind to the CD228 protein with relatively high affinity.

### Example 4: Detection of Binding Specificity of Rabbit Anti-CD228 Monoclonal Antibodies to CD228 Protein at Protein Level by Western Blot

### Experimental method:

A protein was extracted from the A375 cell line using PierceTM RIPA Buffer (purchased from thermo scientific, Cat. No.: WD321069) as an experimental sample, and a CD228-his protein was used as a control sample. The samples were loaded at loading amounts of 6 µg and 600 ng, respectively, for electrophoresis. After electrophoresis, the membrane was transferred by the full wet transfer at 400 mA for 20 min, taken out, and blocked with 5% skim milk powder for 1 h. The membrane was incubated with R546, R526, and CA2204 antibodies, respectively, at 4 °C overnight.

The next day, the membrane was incubated with the corresponding secondary antibody for 1 h, followed by incubation with a chemiluminescent substrate. Imaging and exposure were performed using a gel imager.

### Experimental results:

The results are shown in FIG. 8. The R546, R526, and CA2204 antibodies could accurately bind to the band at the same position as the control CD228-his protein in A375 cells, indicating that the above three antibodies all have relatively good specificity.

### Example 5: Immunohistochemistry Experimental Method for Detecting CD228 in Paraffin-Embedded Tissue Sections with Rabbit Anti-CD228 Monoclonal Antibodies

### 1. Experimental method

### 1) Preparation of paraffin sections:

The obtained target tissues were fixed in 4% paraformaldehyde at 4 °C overnight. The tissues were first dehydrated using a graded ethanol series, then immersed in xylene to ensure thorough dehydration. Subsequently, the tissues were immersed in a mixed solution of xylene and paraffin and treated at 65 °C for 30 min to allow wax infiltration. Finally, the tissues were completely immersed in paraffin at 65 °C for 45 min for embedding.

### 2) Immunohistochemistry operational procedure as follows:

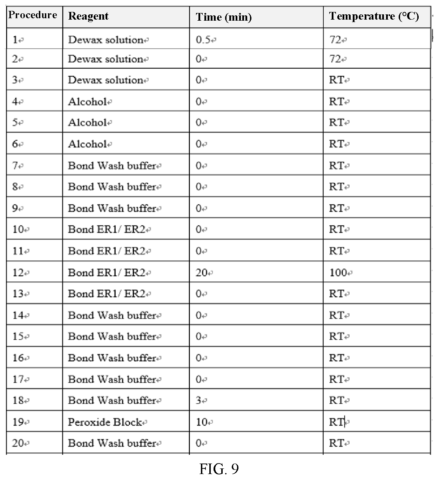

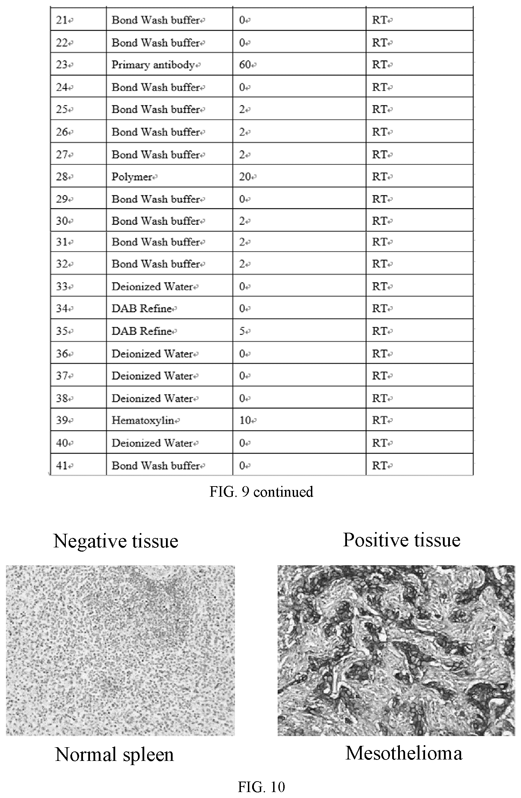

The Leica BOND^{™} fully automated IHC and ISH staining system (model: BOND-MAX) was used. The staining procedure is shown in FIG. 9. FIG. 9 is a flowchart of the Leica BOND^{™} fully automated IHC and ISH staining system.

### 3) Result determination method:

Negative control tissue: A normal human spleen tissue section theoretically lacking CD228 expression was used as the negative control tissue in the experiment. The tissue should show no specific binding to either the irrelevant antibody or the anti-CD228 antibodies. Positive control tissue: A human mesothelioma tissue section with high CD228 expression was used as the positive control tissue in the experiment. The tissue should show no specific binding to the irrelevant antibody but should show specific binding to the anti-CD228 antibodies. The use of these two types of tissues described above in the experiment serves as a quality control standard within the system, ensuring the authenticity and reliability of the results in batch experiments.

### 4) Result evaluation criteria:

Step 1: Analysis of control tissues
   - First, perform staining analysis on the positive and negative control tissue samples.
   - Ensure that the positive control shows the expected specific staining, while the negative control shows no staining or only very weak non-specific staining.
Step 2: Verification of staining specificity
   - Observe the human tissue section treated with the irrelevant antibody, which should theoretically show no staining.
   - If staining occurs, compare it with samples treated with the specific antibody.
   - Results are considered specifically positive only if the area corresponding to the stained area in the specific antibody-treated group shows no staining in the irrelevant antibody group.
Step 3: Quantification of CD228 expression level
   - Evaluate the staining intensity on the tumor cell membrane.
   - Calculate the proportion of tumor cells with membrane staining within the tumor area.
   - Multiply the staining intensity by the proportion of tumor cells with membrane staining to obtain a CD228 expression level score.
   - In this process, ignore staining of immune cells and focus on the evaluation of tumor cells.

### Example 6: Exploration of Experimental Conditions for Immunohistochemistry Detection of Rabbit Anti-CD228 Monoclonal Antibodies

### 1. Experimental method:

### 1) Screening of CD228 positive and negative tissues

A commercial CD228/MFI2 antibody (Novusbio NBP1-85777) was selected as the control antibody at a concentration of 1 µg/mL for the screening of positive and negative tissues; tissue sections were purchased from Shanghai Outdo.

### 2) Selection of commercial antibodies

Under the experimental conditions described in Example 5, the staining effects of 3 commercial antibodies were compared. The three commercial antibodies were as follows: 1. Abcam, Cat. No.: ab236732, a working concentration of 7 µg/mL; 2. R&D system, Cat. No.: MAB8175, working concentrations of 1 µg/mL and 2 µg/mL; 3. Novusbio, Cat. No.: NBP1-85777, 1 µg/mL.

### 3) Exploration of experimental conditions for immunohistochemistry

The optimal staining conditions were explored to obtain clear staining effects in mesothelioma tissues. 3 candidate anti-CD228 antibodies (R546, R526, CA2204) prepared in Example 1 of the present application were used to explore the conditions on the LEICA BOND^{™} fully automated IHC and ISH staining system (model: BOND-MAX) for human mesothelioma tissues (purchased from Shanghai Outdo). The primary antibody in the negative control group was replaced with an irrelevant antibody. The antibody concentration and DAB staining time were used as two variables for exploration.

Antibody concentration gradient setup: Three different antibody working concentrations were set: low concentration (e.g., 1 µg/mL), medium concentration (e.g., 5 µg/mL), and high concentration (e.g., 10 µg/mL). It was ensured that staining with antibodies at each concentration gradient was performed on the same batch of mesothelioma tissue samples to minimize the impact of batch differences on experimental results.

DAB staining time adjustment: For each antibody concentration gradient, DAB staining was performed twice, once for 1 min and once for 5 min, to observe the staining effects.

### 2. Experimental results

Result 1: A normal human spleen tissue section was selected as the negative tissue, and the tissue showed no specific binding to the anti-CD228 antibodies; a human mesothelioma tissue section was selected as the positive tissue, and the tissue showed specific binding to the anti-CD228 antibodies. The screening results for positive and negative tissues are shown in FIG. 10.

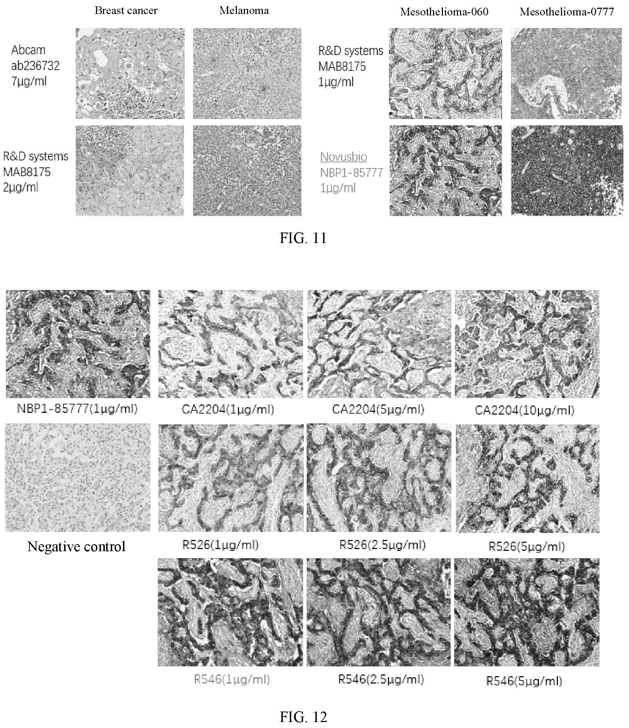

Result 2: As can be seen from the comparison of the staining effects of different commercial antibodies in different tissues shown in FIG. 11, the commercial antibody NBP1-85777 had a better staining effect and could be used as the subsequent control antibody.

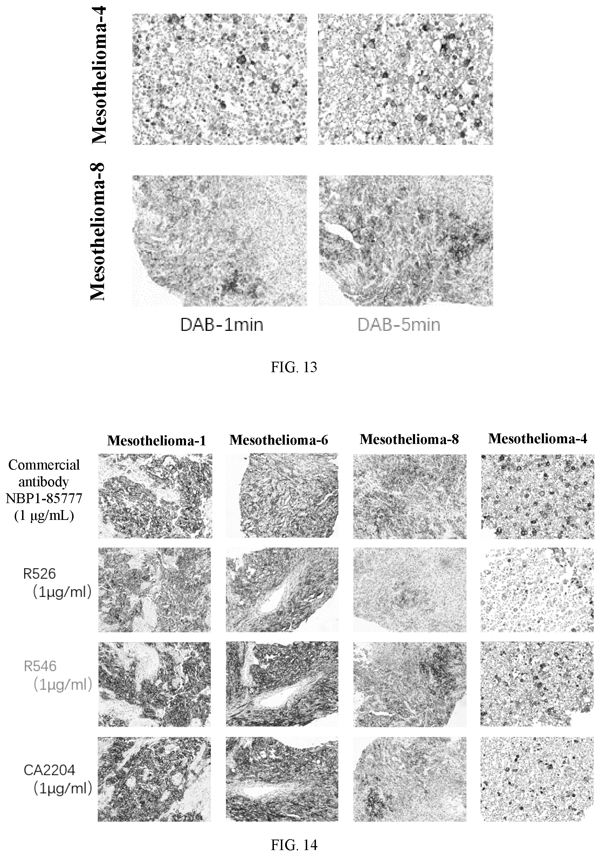

Result 3: The staining results are shown in FIG. 12, which determined that the primary antibody concentration for on-machine experiments was 1 µg/mL; the DAB staining results are shown in FIG. 13, indicating that a DAB staining time of 5 min provided better effects of the R546 antibody in mesothelioma tissues.

### Example 7: Comparison of Staining Effects of Candidate Anti-CD228 Antibody and Commercial Antibody in Different Tissues

### 1. Experimental method:

The candidate anti-CD228 antibody was set as the experimental group, and the commercial CD228/MFI2 antibody (purchased from Novusbio, NBP1-85777) screened in Example 6 was the control group. The tissues obtained in Example 6 were used as the positive and negative controls: a normal human spleen tissue section as the negative tissue, and a human mesothelioma tissue section as the positive tissue. The immunohistochemistry detection method referred to the method in Example 5. The DAB staining time was 5 min, and the primary antibody concentration was 1-4 µg/mL.

### 2. Experimental results:

### Result 1: comparison of staining effects of the candidate anti-CD228 antibody and the commercial antibody NBP1-85777 in mesothelioma tissues

The antibodies were used for staining in 4 different mesothelioma tissues (purchased from Shanghai Outdo) at 1 µg/mL. The immunohistochemistry results are shown in FIG. 14. The results showed that the staining intensity of the R546 antibody was more consistent with that of the commercial antibody NBP1-85777.

### Result 2: comparison of staining in individual tissue sections of mesothelioma, colon cancer, and cervical cancer between R546 and NBP1-85777.

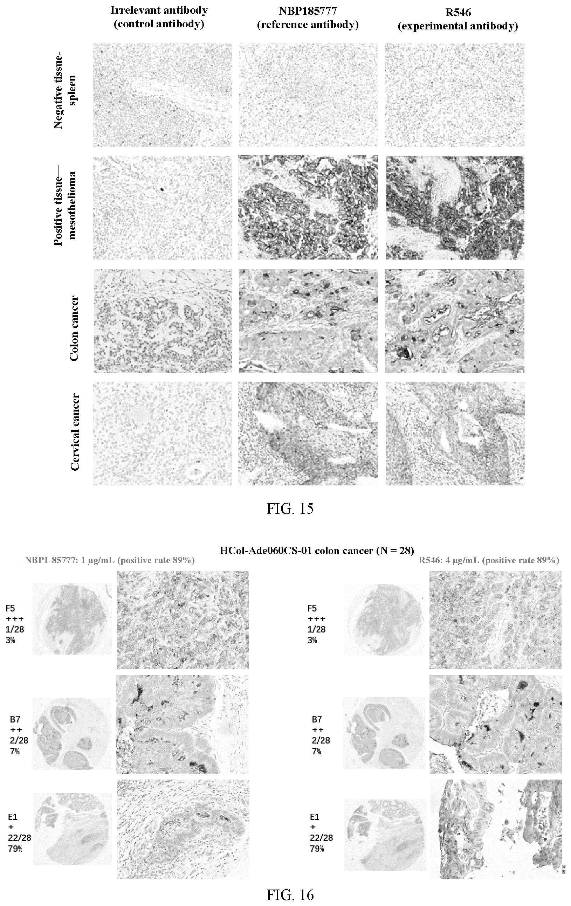

The candidate antibody R546 (at a working concentration of 1 µg/mL), the commercial anti-CD228 monoclonal antibody NBP1-85777 (at a working concentration of 1 µg/mL), and a normal irrelevant antibody (at a working concentration of 1 µg/mL) were used as the primary antibodies, respectively. These were applied to normal human spleen tissue (CD228-negative tissue), human mesothelioma tissue (CD228-positive tissue), colon cancer tissue, and cervical cancer tissue (all tissues purchased from Shanghai Outdo Biotech) for detection using the LEICA BOND^{™} fully automated IHC and ISH staining system (model: BOND-MAX). Acid retrieval was selected for antigen retrieval. The immunohistochemistry results are shown in FIG. 15, and the staining result evaluation is shown in Table 10.

**Table 10. Comparison of staining effects of CD228-2204 and commercial antibody on paraffin-embedded tissue sections**

| Antibody type | Irrelevant antibody | NBP1-85777 | R546 |
|---|---|---|---|
| Working concentration | 1 µg/mL | 1 µg/mL | 1 µg/mL |
| Spleen tissue section | No (-) | No (-) | No (-) |
| Mesothelioma tissue section | No (-) | Strong (+++) | Strong (+++) |
| Colon cancer tissue section | No (-) | Medium (++) | Medium (++) |
| Cervical cancer tissue section | No (-) | Weak (+) | Weak (+) |

According to the observation of the staining results, the results are as follows:
(1) Specificity verification: In the CD228-negative spleen tissue sections, the self-made antibody R546, the commercial antibody NBP1-85777, and the irrelevant antibody all failed to induce a staining reaction. This result indicates that the antibody R546 has high specificity for the CD228-negative sample and can effectively distinguish between tissues with and without CD228 expression.
(2) Consistency of staining effect: The self-made antibody R546 specifically stained the CD228-positive mesothelioma tissue at a working concentration of 1 µg/mL, and its staining intensity matched that of the commercial antibody NBP1-85777 at the same concentration. This indicates that the self-made antibody R546 possesses staining effects equivalent to those of the commercial antibody NBP1-85777 in practical applications and can serve as an effective substitute.
(3) Staining performance in other cancer tissues: In colon cancer and cervical cancer tissues, both the self-made antibody R546 and the commercial antibody NBP1-85777 exhibited staining to varying degrees. This indicates that both antibodies have relatively good staining effects in these cancer types, with comparable staining intensity, further confirming the applicability and reliability of the self-made antibody R546.

Result 3: Comparison of staining in colon cancer tissue chips between R546 and NBP1-85777 Colon cancer tissue chips containing 28 patient tissue samples (purchased from Shanghai Outdo, Cat. No.: Hcol-ade060CS-01) were detected using the LEICA BOND^{™} fully automated IHC and ISH staining system (model: BOND-MAX) with R546 (4 µg/mL) and NBP1-85777 (1 µg/mL), respectively. The immunohistochemistry results are shown in FIG. 16, and the staining result evaluation is shown in Table 11. The staining results showed that the staining intensity and positive rate of R546 and NBP1-85777 on the colon cancer tissue chips were consistent.

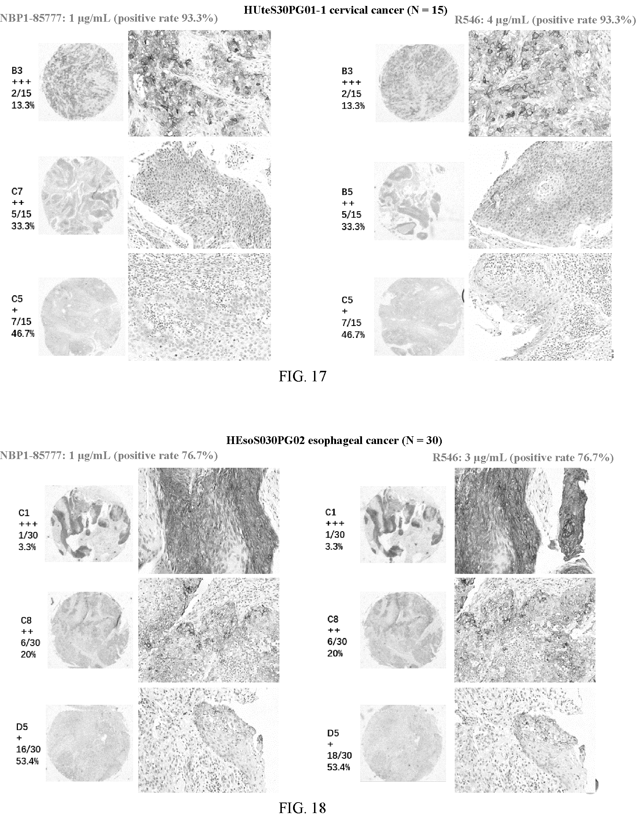

Result 4: Comparison of staining in cervical cancer tissue chips between R546 and NBP1-85777 Cervical cancer tissue chips containing 15 patient tissue samples (purchased from Shanghai Outdo, HUteS30PG01-1) were detected using the LEICA BOND^{™} fully automated IHC and ISH staining system (model: BOND-MAX) with R546 (4 µg/mL) and NBP1-85777 (1 µg/mL), respectively. The immunohistochemistry results are shown in FIG. 17, and the staining result evaluation is shown in Table 11. The staining results showed that the staining intensity and positive rate of R546 and NBP1-85777 on the cervical cancer tissue chips were consistent.

Result 5: Comparison of staining in esophageal cancer tissue chips between R546 and NBP1-85777 Esophageal cancer tissue chips containing 30 patient tissue samples (purchased from Shanghai Outdo, HEsoS030PG02) were detected using the LEICA BOND^{™} fully automated IHC and ISH staining system (model: BOND-MAX) with R546 (3 µg/mL) and NBP1-85777 (1 µg/mL), respectively. The immunohistochemistry results are shown in FIG. 18, and the staining result evaluation is shown in Table 11. The staining results showed that the staining intensity and positive rate of R546 and NBP1-85777 on the esophageal cancer tissue chips were consistent.

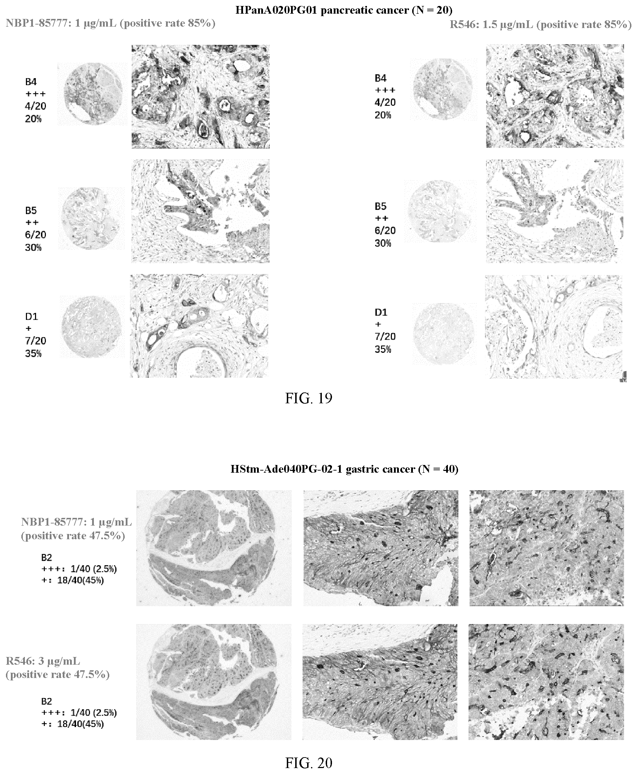

Result 6: Comparison of staining in pancreatic cancer tissue chips between R546 and NBP1-85777 Pancreatic cancer tissue chips containing 20 patient tissue samples (purchased from Shanghai Outdo, HPanA020PG01) were detected using the LEICA BOND^{™} fully automated IHC and ISH staining system (model: BOND-MAX) with R546 (1.5 µg/mL) and NBP1-85777 (1 µg/mL), respectively. The immunohistochemistry results are shown in FIG. 19, and the staining result evaluation is shown in Table 11. The staining results showed that the staining intensity and positive rate of R546 and NBP1-85777 on the pancreatic cancer tissue chips were consistent.

Result 7: Comparison of staining in gastric cancer tissue chips between R546 and NBP1-85777 Gastric cancer tissue chips containing 40 patient tissue samples (purchased from Shanghai Outdo, HStm-Ade040PG-02-2) were detected using the LEICA BOND^{™} fully automated IHC and ISH staining system (model: BOND-MAX) with R546 (3 µg/mL) and NBP1-85777 (1 µg/mL), respectively. The immunohistochemistry results are shown in FIG. 20, and the staining result evaluation is shown in Table 11. The staining results showed that the staining intensity and positive rate of R546 and NBP1-85777 on the gastric cancer tissue chips were consistent.

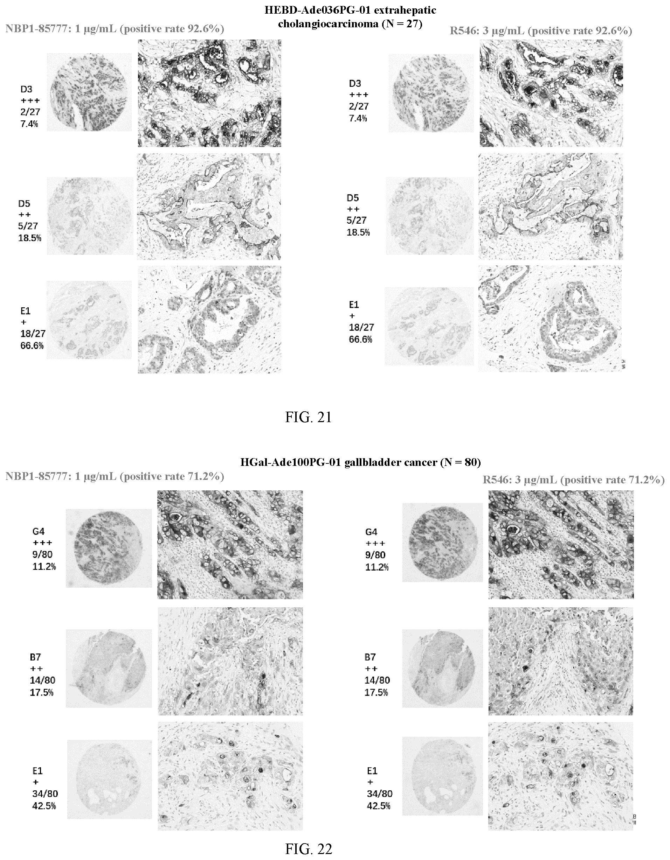

Result 8: Comparison of staining in extrahepatic cholangiocarcinoma tissue chips between R546 and NBP1-85777
Extrahepatic cholangiocarcinoma tissue chips containing 40 patient tissue samples (purchased from Shanghai Outdo, HEBD-ade036PG-01) were detected using the LEICA BOND^{™} fully automated IHC and ISH staining system (model: BOND-MAX) with R546 (3 µg/mL) and NBP1-85777 (1 µg/mL), respectively. The immunohistochemistry results are shown in FIG. 21, and the staining result evaluation is shown in Table 11. The staining results showed that the staining intensity and positive rate of R546 and NBP1-85777 on the extrahepatic cholangiocarcinoma tissue chips were consistent.

Result 9: Comparison of staining in gallbladder cancer tissue chips between R546 and NBP1-85777 Gallbladder cancer tissue chips containing 80 patient tissue samples (purchased from Shanghai Outdo, HGal-Ade100PG-01) were detected using the LEICA BOND^{™} fully automated IHC and ISH staining system (model: BOND-MAX) with R546 (3 µg/mL) and NBP1-85777 (1 µg/mL), respectively. The immunohistochemistry results are shown in FIG. 22, and the staining result evaluation is shown in Table 11. The staining results showed that the staining intensity and positive rate of R546 and NBP1-85777 on the gallbladder cancer tissue chips were consistent.

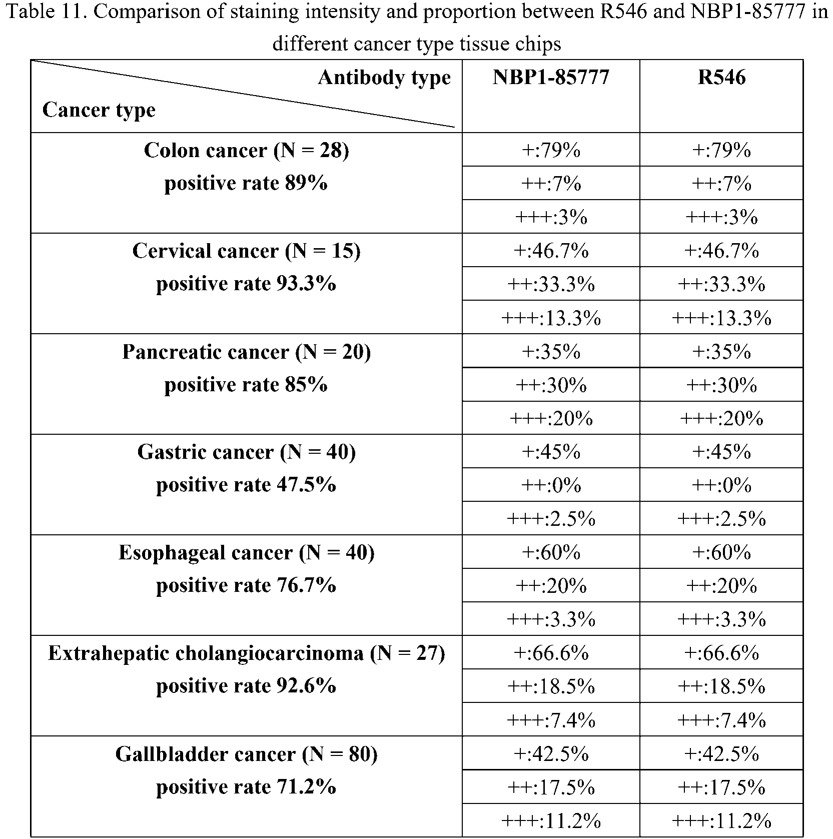

The data in Table 11 indicate that the staining intensity and positive rate of R546 and NBP1-85777 were consistent in the above 7 cancer types (colon cancer, cervical cancer, pancreatic cancer, gastric cancer, esophageal cancer, extrahepatic cholangiocarcinoma, and gallbladder cancer).

Result 10: The staining of the two antibodies, R546 and NBP1-85777, was validated in different cancer tissues. The experimental method was the same as in Example 5. Specifically, the company performed immunohistochemical staining in individual tissue sections of gallbladder cancer, cholangiocarcinoma, esophageal cancer, pancreatic cancer, and gastric cancer, and compared the staining effects of these two antibodies.

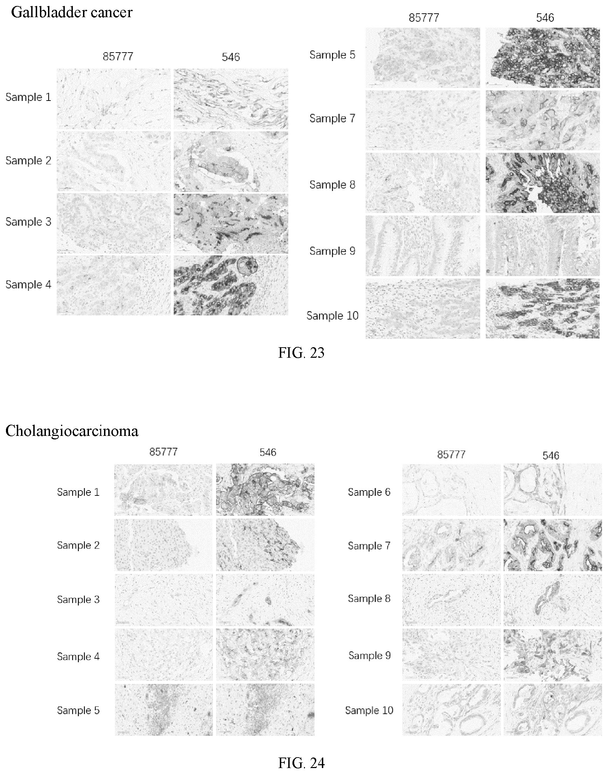

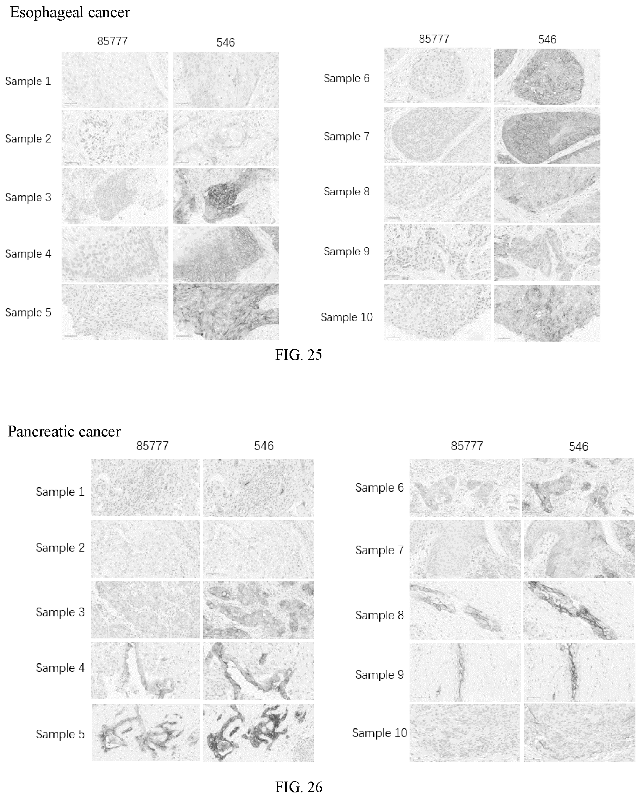

The candidate antibody R546 (at a working concentration of 1 µg/mL) and the commercial anti-CD228 monoclonal antibody NBP1-85777 (at a working concentration of 1 µg/mL) were used as the primary antibodies, respectively. These were applied to individual tissue section samples from 10 patients for each cancer type: gallbladder cancer, cholangiocarcinoma, esophageal cancer, pancreatic cancer, and gastric cancer tissues (all tissues from ACRODiagnostics) for detection using the LEICA BOND^{™} fully automated IHC and ISH staining system (model: BOND-III). The immunohistochemistry results are shown in FIGs. 23-27, and the staining result evaluation is shown in Table 12. The staining results showed that in the individual tissue sections of the above five cancer types (gallbladder cancer, cholangiocarcinoma, esophageal cancer, pancreatic cancer, and gastric cancer), the staining intensity and staining sensitivity of R546 were significantly better than those of NBP1-85777.

**Table 12. Comparison of staining in individual tissue sections of gallbladder cancer, cholangiocarcinoma, esophageal cancer, pancreatic cancer, and gastric cancer between R546 and NBP1-85777**

| Cancer type | 85777 (1µg/mL) | | | | 546 (1µg/mL) | | | |
|---|---|---|---|---|---|---|---|---|
| | +++ | ++ | + | Positive rate% | +++ | ++ | + | Positive rate% |
| Gallbladder cancer (n = 10) | 0 | 0 | 4 | 40% | 4 | 3 | 2 | 90% |
| Cholangiocarcinoma (n = 10) | 0 | 0 | 6 | 60% | 0 | 3 | 7 | 100% |
| Esophageal cancer (n = 10) | 0 | 0 | 0 | 0% | 0 | 3 | 7 | 100% |
| Pancreatic cancer (n = 10) | 0 | 0 | 6 | 60% | 0 | 0 | 8 | 80% |
| Gastric cancer (n = 10) | 0 | 0 | 4 | 40% | 0 | 1 | 6 | 70% |

The data in the table indicate that in the individual tissue sections of the above five cancer types (gallbladder cancer, cholangiocarcinoma, esophageal cancer, pancreatic cancer, and gastric cancer), the positive rate of R546 was significantly higher than that of NBP1-85777. This demonstrates that the R546 antibody of the present disclosure can replace the existing commercial antibody for immunohistochemistry detection of the CD228 target.

Based on the experiments described above, it can be seen that the R546 antibody has higher sensitivity and potentially better diagnostic efficacy in the immunohistochemistry detection of these specific cancer types. The high positive rate of the R546 antibody may also imply that it has better specificity, enabling more accurate distinction between cancer cells and normal cells, which is crucial for early discovery and treatment selection of cancer. Furthermore, the high sensitivity of the R546 antibody may help identify cases with low or heterogeneous expression that might be missed by the NBP1-85777 antibody.

### Example 8: Detection of Binding Specificity of R546 by ELISA

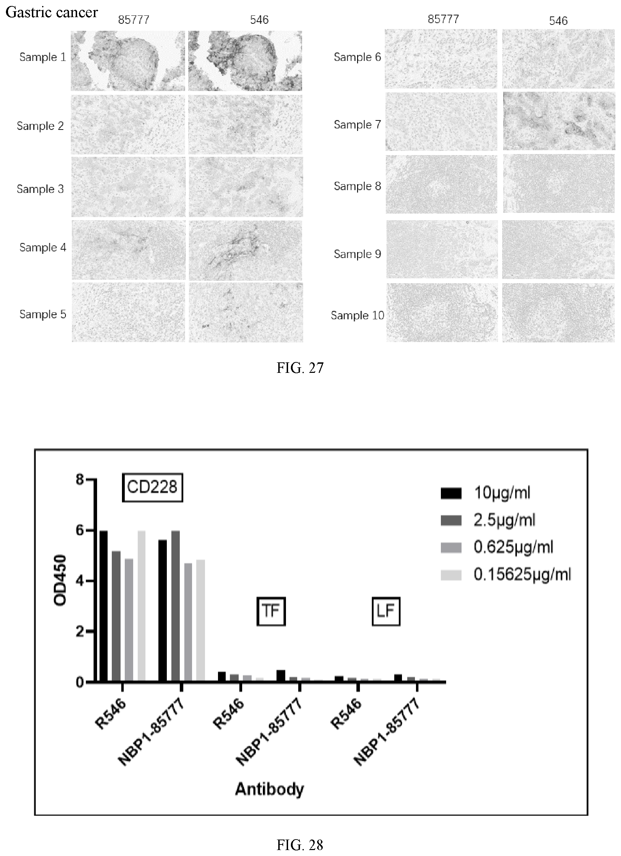

The CD228 protein, transferrin (TF), and lactoferrin (LF) were diluted to 0.2 µg/mL with CBS and added to a plate at 100 µL/well, and the plate was incubated at 4 °C overnight. A blocking solution was added, and the plate was blocked at 37 °C for 1 h and washed. R546 and NBP1-85777 were separately subjected to a dilution (starting from 10 µg/mL, four-fold dilution, 8 gradients) with PBST and added to the plate at 100 µL/well, and the plate was incubated at 37 °C for 1 h and washed. The enzyme-labeled secondary antibody, Goat pAb to Rb IgG (HRP), was added at 100 µL/well, and the plate was incubated at 37 °C for 1 h and washed. 100 µL of a TMB chromogenic solution was added for color development for 10 min, and 2 M sulfuric acid was added to stop the reaction. The OD450nm values were read using a microplate reader. The OD450 values were plotted against the antibody concentrations, and the results are shown in FIG. 28. The results showed that both R546 and NBP1-85777 bound only to the CD228 protein, and had substantially no binding to transferrin and lactoferrin of the same family, indicating that the antibodies had good selectivity and specificity.

### Example 9: Detection of Binding Specificity of R546 by Bio-Layer Interferometry

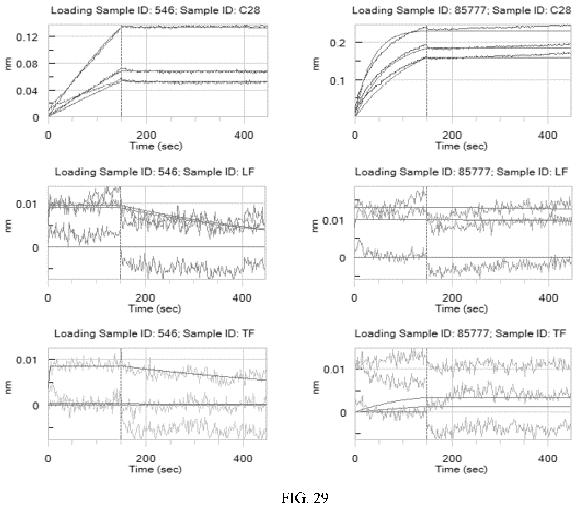

The R546 and NBP1-85777 antibodies were coupled to ProA (Fortebio, Cat. No.: 18-5010) chips on the Fortebio RED96 instrument at a concentration of 4 µg/mL, with a threshold set to 1.5 nm. The CD228 protein, transferrin (TF), and lactoferrin (LF) were subjected to a 2-fold gradient dilution starting from a concentration of 100 nM with PBST to obtain 4 concentrations. The association lasted for 150 s, the dissociation lasted for 300 s, the equilibrium lasted for 60 s, and the probe regeneration lasted for 15 s. Kinetic parameters, including the KD value, were calculated using the instrument's supporting software. The detection data are shown in Table 13, and the corresponding affinity detection spectra are shown in FIG. 29.

**Table 13. KD of R546 and NBP1-85777 binding to CD228 and other proteins of same family**

| Ab | Ag | KD (M) | Response |
|---|---|---|---|
| R546 | C28 | 1.33E-09 | 0.1278 |
| | TF | no binding | 0.0006 |
| | LF | no binding | 0.0129 |
| NBP1-85777 | C28 | <1.0E-12 | 0.2389 |
| | TF | no binding | 0.0063 |
| | LF | no binding | 0.0174 |

The data in the table show that both R546 and NBP1-85777 bound only to the CD228 protein, and had no binding to transferrin and lactoferrin of the same family, indicating that the antibodies had good selectivity and specificity.

### Example 10: Detection of Placement Stability of R546 Antibody

The R546 antibody was stored in a constant temperature environment at 25 °C and taken out on day 30; the R546 antibody was stored in a constant temperature environment at 40 °C and taken out on day 0, day 7, and day 14, respectively. The above antibodies were subjected to size/volume exclusion chromatography (SEC), reduced capillary electrophoresis with sodium dodecyl sulfate (rCE-SDS), and imaged capillary isoelectric focusing (icIEF) detection to investigate sample stability. SEC evaluates changes in antibody polymers and small molecule content through separation by molecular size; rCE-SDS evaluates the structural stability of the antibody by analyzing the percentage content of light and heavy chains; and icIEF characterizes the charge heterogeneity of the antibody through the distribution of isoelectric points. The numerical trends and peak characteristics of these techniques provide a comprehensive evaluation of antibody stability. The detection data are shown in Tables 14, 15, and 16.

**Table 14. Changes in icIEF of R546 antibody after storage at different temperatures for different days**

| Sample No. | Isoelectric point range | Main peak isoelectric point | Percentage content (%) | | |
|---|---|---|---|---|---|
| | | | Acidic peak | Main peak | Basic peak |
| CD228-R546-0d | 5.61-6.61 | 5.83 | 32.7 | 46.1 | 21.2 |
| CD228-R546-40°C-7d | 5.61-6.61 | 5.83 | 33.8 | 43.6 | 22.7 |
| CD228-R546-40°C-14d | 5.61-6.61 | 5.83 | 35.0 | 41.6 | 23.4 |
| CD228-R546-25°C-30d | 5.61-6.60 | 5.83 | 31.9 | 46.1 | 22.0 |

**Table 15. Changes in rCE-SDS of R546 antibody after storage at different temperatures for different days**

| Sample No. | Percentage content (%) | | |
|---|---|---|---|
| | Light chain | Heavy chain | Light chain + heavy chain |
| CD228-R546-0d | 27.8 | 72.0 | 99.8 |
| CD228-R546-40°C-7d | 27.4 | 72.4 | 99.8 |
| CD228-R546-40°C-14d | 27.6 | 72.2 | 99.8 |
| CD228-R546-25°C-30d | 27.3 | 72.5 | 99.8 |

**Table 16. Changes in SEC of R546 antibody after storage at different temperatures for different days**

| Sample No. | Percentage content (%) | | |
|---|---|---|---|
| | Polymer | Monomer | Small molecule |
| CD228-R546-0d | 0.4 | 99.5 | 0.0 |
| CD228-R546-40°C-7d | 0.7 | 99.2 | 0.1 |
| CD228-R546-40°C-14d | 0.7 | 99.0 | 0.3 |
| CD228-R546-25°C-30d | 0.4 | 99.5 | 0.1 |

Under high temperature (40 °C) conditions, the icIEF (isoelectric focusing electrophoresis) results showed a slight decrease in the main peak content, a slight increase in the acidic peak content, and a slight increase in the basic peak content. The rCE-SDS (capillary electrophoresis-sodium dodecyl sulfate polyacrylamide gel electrophoresis) results showed no significant changes in the percentage content of light chain %, heavy chain %, and (light chain + heavy chain) %. The SEC (size exclusion chromatography) purity showed no significant changes. Under high temperature (25 °C) conditions, no significant changes were observed in all detection results for the R546 antibody. The above results indicate good stability of the antibody.

In summary, the self-made R546 monoclonal antibody exhibits the highest binding sensitivity to the CD228 protein, possesses relatively strong affinity, and specifically binds only to the CD228 protein without binding to other proteins in the same family. Furthermore, based on the immunohistochemistry results, comprehensive staining effects on both individual tissue sections and tissue chips of different cancers demonstrate that the self-made antibody R546 has superior staining sensitivity and positive rates compared to the commercial antibody NBP1-85777. Therefore, we conclude that R546 can replace the commercial antibody as a diagnostic antibody suitable for immunohistochemistry detection.

## Claims

1. An antibody or an antigen-binding fragment thereof specifically binding to a CD228 protein, comprising the following 3 light chain complementarity determining regions and 3 heavy chain complementarity determining regions, wherein
the 3 light chain complementarity determining regions of the antibody or the antigen-binding fragment thereof comprise an LCDR1 set forth in SEQ ID NO: 2, an LCDR2 set forth in SEQ ID NO: 3, and an LCDR3 set forth in SEQ ID NO: 4; and the 3 heavy chain complementarity determining regions comprise an HCDR1 set forth in SEQ ID NO: 6, an HCDR2 set forth in SEQ ID NO: 7, and an HCDR3 set forth in SEQ ID NO: 8;
the 3 light chain complementarity determining regions of the antibody or the antigen-binding fragment thereof comprise an LCDR1 set forth in SEQ ID NO: 10, an LCDR2 set forth in SEQ ID NO: 11, and an LCDR3 set forth in SEQ ID NO: 12; and the 3 heavy chain complementarity determining regions comprise an HCDR1 set forth in SEQ ID NO: 14, an HCDR2 set forth in SEQ ID NO: 15, and an HCDR3 set forth in SEQ ID NO: 16; or
the 3 light chain complementarity determining regions of the antibody or the antigen-binding fragment thereof comprise an LCDR1 set forth in SEQ ID NO: 18, an LCDR2 set forth in SEQ ID NO: 19, and an LCDR3 set forth in SEQ ID NO: 20; and the 3 heavy chain complementarity determining regions comprise an HCDR1 set forth in SEQ ID NO: 22, an HCDR2 set forth in SEQ ID NO: 23, and an HCDR3 set forth in SEQ ID NO: 24.

2. The antibody or the antigen-binding fragment thereof according to claim 1, comprising a light chain variable region having at least 95%, 96%, 97%, 98%, 99%, or 100% identity to the amino acid sequence set forth in SEQ ID NO: 1, and a heavy chain variable region having at least 95%, 96%, 97%, 98%, 99%, or 100% identity to the amino acid sequence set forth in SEQ ID NO: 5;
comprising a light chain variable region having at least 95%, 96%, 97%, 98%, 99%, or 100% identity to the amino acid sequence set forth in SEQ ID NO: 9, and a heavy chain variable region having at least 95%, 96%, 97%, 98%, 99%, or 100% identity to the amino acid sequence set forth in SEQ ID NO: 13; or
comprising a light chain variable region having at least 95%, 96%, 97%, 98%, 99%, or 100% identity to the amino acid sequence set forth in SEQ ID NO: 17, and a heavy chain variable region having at least 95%, 96%, 97%, 98%, 99%, or 100% identity to the amino acid sequence set forth in SEQ ID NO: 21.

3. The antibody or the antigen-binding fragment thereof according to claim 1 or 2, comprising a heavy chain constant region having at least 95%, 96%, 97%, 98%, 99%, or 100% identity to the amino acid sequence set forth in SEQ ID NO: 25, and comprising a light chain constant region having at least 95%, 96%, 97%, 98%, 99%, or 100% identity to the amino acid sequence set forth in SEQ ID NO: 26.

4. An antibody conjugate, wherein the antibody conjugate consists of the antibody or the antigen-binding fragment thereof specifically binding to a CD228 protein according to any one of claims 1-3 and at least one detectable label.

5. A nucleic acid, wherein the nucleic acid encodes the antibody or the antigen-binding fragment thereof specifically binding to a CD228 protein according to any one of claims 1-3.

6. A kit for detecting or quantifying CD228, comprising the antibody or the antigen-binding fragment thereof specifically binding to a CD228 protein according to any one of claims 1-3, or the antibody conjugate according to claim 4, wherein optionally, the kit further comprises a relevant buffer solution for detection or quantification.

7. A kit for diagnosing a disease or evaluating a therapeutic effect on a disease, comprising the antibody or the antigen-binding fragment thereof specifically binding to a CD228 protein according to any one of claims 1-3, or the antibody conjugate according to claim 4, wherein the disease is a CD228-related disease;
preferably, the CD228-related disease comprises one or more of melanoma, lung cancer, gastric cancer, colon cancer, mesothelioma, pancreatic cancer, breast cancer, esophageal cancer, cervical cancer, gallbladder cancer, and cholangiocarcinoma.

8. Use of the antibody or the antigen-binding fragment thereof specifically binding to a CD228 protein according to any one of claims 1-3, the antibody conjugate according to claim 4, or the nucleic acid according to claim 5 in the manufacture of a kit for detecting and/or quantifying whether a cell or tissue expresses CD228, wherein
preferably, the kit is an immunohistochemistry detection kit.

9. Use of the antibody or the antigen-binding fragment thereof specifically binding to a CD228 protein according to any one of claims 1-3 in the manufacture of a kit for diagnosing a disease, wherein the disease is a CD228-related disease;
preferably, the kit is an immunohistochemistry detection kit;
preferably, the CD228-related disease comprises one or more of melanoma, lung cancer, gastric cancer, colon cancer, mesothelioma, pancreatic cancer, breast cancer, esophageal cancer, cervical cancer, gallbladder cancer, and cholangiocarcinoma.

10. Use of the antibody or the antigen-binding fragment thereof specifically binding to a CD228 protein according to any one of claims 1-3, the antibody conjugate according to claim 4, or the nucleic acid according to claim 5 in the manufacture of a kit for evaluating a therapeutic effect on a CD228-related disease, wherein the disease is a CD228-related disease;
preferably, the CD228-related disease comprises one or more of melanoma, lung cancer, gastric cancer, colon cancer, mesothelioma, pancreatic cancer, breast cancer, esophageal cancer, cervical cancer, gallbladder cancer, and cholangiocarcinoma.
